(19) Europäisches Patentamt
European Patent Office
Office européen des brevets

(11) **EP 4 484 005 A1**

(12) **EUROPEAN PATENT APPLICATION**
published in accordance with Art. 153(4) EPC

(43) Date of publication:
01.01.2025 Bulletin 2025/01

(21) Application number: 23780832.4

(22) Date of filing: 30.03.2023

(51) International Patent Classification (IPC):
*B01J 29/40* (2006.01)    *C01B 39/36* (2006.01)
*C07C 1/20* (2006.01)    *C07C 15/08* (2006.01)
*C07B 61/00* (2006.01)

(52) Cooperative Patent Classification (CPC):
B01J 29/40; C01B 39/36; C07C 1/20; C07C 15/08;
C07B 61/00; Y02P 20/52

(86) International application number:
PCT/JP2023/013080

(87) International publication number:
WO 2023/190824 (05.10.2023 Gazette 2023/40)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 31.03.2022 JP 2022058510

(71) Applicants:
• KAWASAKI JUKOGYO KABUSHIKI KAISHA
Kobe-shi, Hyogo 650-8670 (JP)
• OSAKA UNIVERSITY
Suita-shi
Osaka 565-0871 (JP)

(72) Inventors:
• YONEDA, Koji
Kobe-shi, Hyogo 650-8670 (JP)
• KITO, Shintaro
Kobe-shi, Hyogo 650-8670 (JP)
• WARATANI, Yusuke
Kobe-shi, Hyogo 650-8670 (JP)
• TANIYAMA, Noriyuki
Kobe-shi, Hyogo 650-8670 (JP)
• NISHIYAMA, Norikazu
Suita-shi
Osaka 565-0871 (JP)
• MIYAKE, Koji
Suita-shi
Osaka 565-0871 (JP)

(74) Representative: Arnold & Siedsma
Bezuidenhoutseweg 57
2594 AC The Hague (NL)

(54) **MANUFACTURING METHOD FOR ZEOLITE CATALYST WITH SILICA COATING, AND ZEOLITE CATALYST**

(57) One aspect of the present invention relates to a method for manufacturing a zeolite catalyst with a silica coating, the method includes at least: mixing a zinc source and an MFI-type zeolite to obtain a zinc-doped zeolite; preparing a mixture liquid containing a structure-directing agent containing tetraethylammonium, a silica source, and water; and adding the mixture liquid to a surface of the zinc-doped zeolite to perform hydrothermal synthesis.

FIG.1

EP 4 484 005 A1

## Description

### Technical Field

The present invention relates to a method for manufacturing a zeolite catalyst with a silica coating and a zeolite catalyst.

### Background Art

In recent years, materialization of carbon dioxide has attracted attention as an alternative method for the manufacture of fossil resources. Specifically, a technique of manufacturing a chemical raw material by reacting carbon dioxide with hydrogen or the like has been studied. For example, it is known to convert carbon dioxide into benzene, methanol, ethylene, xylene, or the like.

As a method for manufacturing an aromatic compound such as xylene, a method for manufacturing an aromatic compound from an alcohol, an olefin, or a paraffin using a crystalline aluminosilicate or a crystalline gallosilicate as a catalyst is known. In the manufacturing method using a catalyst, however, xylene is obtained as a mixture of isomers of a para-form, a meta-form, and an ortho-form, and thus a special separation device or the like is required to separate paraxylene from the isomers. Therefore, a manufacturing method capable of selectively providing paraxylene among xylenes is demanded.

So far, as a technique capable of providing an aromatic hydrocarbon mixture having a high ratio of paraxylene among xylene isomers, there has been reported a method for manufacturing an aromatic hydrocarbon from at least one compound selected from among an alcohol, an olefin, and a paraffin, wherein a reaction is performed in the presence of a catalyst containing a compound obtained by modifying a silicate selected from a crystalline aluminosilicate and a crystalline gallosilicate with silica (Patent Literature 1).

The technique proposed in Patent Literature 1 is reported to be able to obtain an aromatic hydrocarbon mixture having a high ratio of paraxylene among xylene isomers, but it has been found by the studies conducted by the present inventors that the yield of xylene itself is reduced.

### Citation List

### Patent Literature

Patent Literature 1: JP-A-2010-208948

### Summary of Invention

Therefore, a main challenge of the present invention is to provide a zeolite catalyst capable of providing a target compound from among isomers at a high ratio and in a high yield when manufacturing an aromatic compound having multiple isomers, such as xylene, and a method for manufacturing the catalyst.

One aspect of the present invention relates to a method for manufacturing a zeolite catalyst with a silica coating, the method including at least: mixing a zinc source and an MFI-type zeolite to obtain a zinc-doped zeolite; preparing a mixture liquid containing a structure-directing agent containing tetraethylammonium, a silica source, and water; and adding the mixture liquid to a surface of the zinc-doped zeolite to perform hydrothermal synthesis.

A zeolite catalyst according to another aspect of the present invention is characterized by being a zeolite catalyst obtained by the manufacturing method.

### Brief Description of Drawings

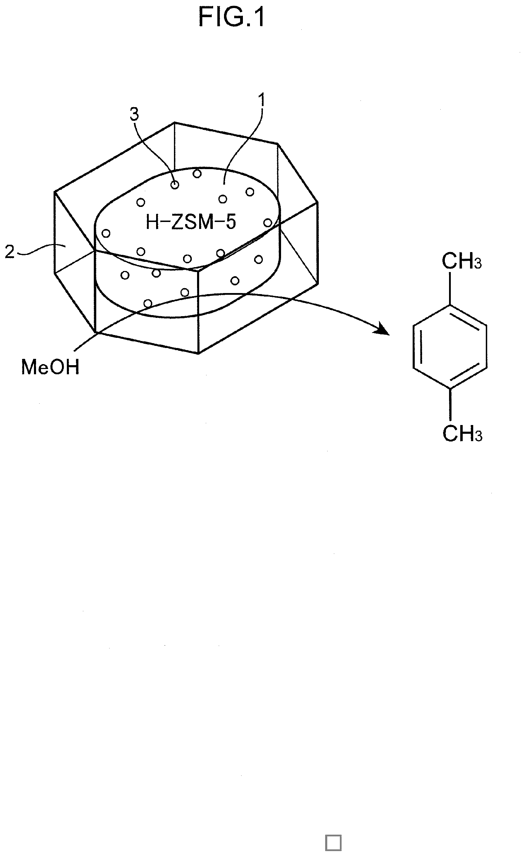

Fig. 1 is a schematic diagram illustrating one embodiment of a zeolite catalyst obtained by the manufacturing method of the present embodiment.

### Description of Embodiments

Hereinafter, an embodiment of the present invention will be specifically described, but the present invention is not limited thereto.

[Method for manufacturing zeolite catalyst]

One embodiment of the present invention is a method for manufacturing a zeolite catalyst with a silica coating, the method including at least: mixing a zinc source and an MFI-type zeolite to obtain a zinc-doped zeolite; preparing a mixture liquid containing a structure-directing agent containing tetraethylammonium, a silica source, and water; and adding the mixture liquid to a surface of the zinc-doped zeolite to perform hydrothermal synthesis.

Such a manufacturing method can provide a zeolite catalyst with a silica coating thinner than conventional products. The zeolite catalyst obtained in the present embodiment is, as illustrated in Fig. 1, a zeolite catalyst having a core-shell structure in which a surface of a zeolite crystal 1 to be a core is coated with a silica coating 2 to be an inert layer. The zeolite crystal 1 to be a core contains zinc ions 3. The zeolite catalyst can inhibit a reaction on the outer surface owing to having the silica coating 2, so that when an aromatic compound having multiple isomers such as xylene is produced using the zeolite catalyst, a target compound can be obtained at a high ratio from among the isomers. In addition, since the silica coating 2 is a thin film, the diffusion resistance of a generated gas can be reduced, and the catalytic activity can be improved. Therefore, there is an advantage that the target compound can be obtained in a high yield.

In the manufacturing method of the present embodiment, first, a zinc source and an MFI-type zeolite are mixed to obtain a zinc-doped zeolite.

In the present embodiment, an MFI-type zeolite is used as a nucleus (core) of a catalyst. The MFI-type zeolite is a zeolite having an oxygen-10-membered ring structure, and it is particularly preferable to use an aluminosilicate zeolite such as a ZSM-5 type zeolite. It is considered that the use of such an MFI-type zeolite makes it possible to selectively provide a desired compound on the basis of a difference in molecular size from among compounds having multiple isomers. Furthermore, there is another advantage that paraxylene can be obtained with a higher selectivity than orthoxylene and metaxylene among xylenes by use of the MFI-type zeolite.

As the MFI-type zeolite as described above, a commercially available zeolite may be used, or the MFI-type zeolite may be synthesized by a known method.

The zinc source that can be used in the present embodiment is not particularly limited as long as zinc ions can thereby be added to the zeolite, and examples thereof include zinc nitrate, zinc acetate, zinc chloride, and zinc sulfate. Among them, zinc nitrate, zinc chloride, and the like are preferably used from the viewpoint of solubility in an aqueous solution. As the zinc source as described above, a hydrate thereof or the like can also be used.

The ratio at which the zinc source and the MFI-type zeolite are mixed is not particularly limited, but for example, it is preferable to mix them such that the weight ratio of the MFI-type zeolite : the zinc source is about 100 : 1 to 1 : 10. More preferably, they are mixed such that the ratio of the MFI-type zeolite : the zinc source is about 20 : 1 to 1 : 2.

When mixing is performed, for example, the MFI-type zeolite may be added to an aqueous solution containing the zinc source, and the mixture liquid may be stirred at a temperature of 20 to 100°C for about 1 to 24 hours.

Thereafter, filtration, washing, and drying are performed, and then calcination is performed to obtain a zinc-doped zeolite. The methods of the filtration, washing, and drying are not particularly limited, and known methods can be appropriately used. After drying, calcination is performed at about 400 to 600°C for 1 to 24 hours.

Next, a mixture liquid containing a structure-directing agent containing tetraethylammonium, a silica source, and water is prepared.

The structure-directing agent to be used in the present embodiment contains at least tetraethylammonium. In previously reported methods for manufacturing a zeolite catalyst, tetrapropylammonium is used as a structure-directing agent. However, tetrapropylammonium has high basicity and is likely to cause dissolution and nucleation of a core catalyst, so that a silica coating to be a shell layer is formed by coarse nuclei. In that case, the core catalyst cannot be sufficiently coated unless the silica coating is formed multiple times. As a result, the silica coating becomes so thick that the zeolite catalytic activity decreases, and the yield in the synthesis using the catalyst decreases.

In contrast, the use of a structure-directing agent containing tetraethylammonium, which has low basicity, as in the present embodiment makes it possible to control the crystal growth rate when forming a silica coating on the surface of a catalyst, and coat the catalyst with a silica coating that is more uniform and thinner than the conventional method. As a result, it is considered that the zeolite catalytic activity increases, and the yield in synthesis and manufacture using the catalyst is improved.

The structure-directing agent of the present embodiment may further contain a structure-directing agent other than the tetraethylammonium, and may also contain, for example, tetrapropylammonium, triethylamine, ethylenediamine, or the like.

The silica source to be used in the present embodiment is not particularly limited as long as it can coat the zinc-doped zeolite, and may be appropriately selected according to the intended composition of the silica coating. Examples thereof specifically include tetraethyl orthosilicate, tetramethyl orthosilicate, sodium silicate, colloidal silica, and fumed silica.

The silica coating is preferably a crystalline silica coating, and particularly preferably a porous silicalite coating from the viewpoint of diffusibility of a reactant (molecule) and a product (molecule). Therefore, tetraethyl orthosilicate, colloidal silica, fumed silica, and the like can be suitably used as the silica source.

The mixture liquid containing the structure-directing agent containing tetraethylammonium, the silica source, and water is used as a raw material liquid for forming a silica coating on a zinc-doped zeolite. Although the solvent contained in the mixture liquid may be only water, a watersoluble organic solvent such as ethanol, propanol, or glycerin may be contained in the mixture liquid in addition to water in order to increase the solubility of the silica source.

The amounts of the tetraethylammonium and the silica source in the mixture liquid are not particularly limited, and may be appropriately adjusted within a range in which a silica coating can be formed on a zinc-doped zeolite.

The amount of the tetraethylammonium in the mixture liquid is preferably adjusted such that the molar ratio of the silica source : the tetraethylammonium is, for example, about 100 : 1 to 1 : 1. More preferably, the tetraethylammonium is used in such an amount that the molar ratio of the silica source : the tetraethylammonium is about 10 : 1 to 2 : 1.

The amount of the silica source in the mixture liquid is preferably adjusted such that, for example, the weight ratio of the zinc-doped zeolite : the silica source is about 20 : 1 to 1 : 5. More preferably, the silica source is used in such an amount that the molar ratio of the zinc-doped zeolite : the silica source is about 5 : 1 to 1 : 2.

Next, the mixture liquid containing the structure-directing agent containing tetraethylammonium, the silica source, and water is added to a surface of the zinc-doped zeolite. The addition method is not particularly limited, and the zinc-doped zeolite may be immersed in the mixture liquid containing the silica source and the structure-directing agent, or the mixture liquid may be applied to the surface of the zinc-doped zeolite.

Thereafter, hydrothermal synthesis is performed. In the present embodiment, the hydrothermal synthesis is not particularly limited regarding the means for performing it, and can be performed using, for example, a rotary autoclave, a stirring autoclave, a static (non-stirring) autoclave, or the like. Specifically, the zinc-doped zeolite may be placed in an autoclave while being immersed in the mixture liquid, and then subjected to hydrothermal synthesis, or the zinc-doped zeolite having the mixture liquid applied to the surface thereof may be placed in hot water in an autoclave, and then subjected to hydrothermal synthesis.

The temperature of the hydrothermal synthesis is preferably about 100 to 200°C, and more preferably 150 to 190°C. The reaction time of the hydrothermal synthesis is preferably about 1 to 200 hours, and more preferably about 4 to 48 hours.

After performing the hydrothermal synthesis, the resulting synthesized product is washed and dried, and then calcined to provide a zeolite catalyst with a silica coating. The methods of washing and drying are not particularly limited, and known methods may be appropriately used. After the drying, the silica coating is calcined at about 400 to 600°C for 1 to 24 hours.

Through the steps described above, the zeolite catalyst of the present embodiment with a silica coating can be obtained. The zeolite catalyst obtained may be used as received, or the particle size thereof may be adjusted by a known granulation method or the like, as necessary.

In the zeolite catalyst obtained by the manufacturing method of the present embodiment, the weight increase rate of the zeolite catalyst after the formation of the silica coating based on the weight of the zinc-doped zeolite before the formation of the silica coating is preferably 40% or less. This weight increase rate is an index indicating that a thin silica coating is formed in the zeolite catalyst obtained in the present embodiment. The weight increase rate is more preferably 20% or less. The lower limit thereof is not particularly limited, but when the weight increase rate is less than 1%, a sufficient silica coating may not be formed, and therefore the weight increase rate is preferably 1% or more.

The weight increase rate is a value determined by the method shown in Examples described later.

[Zeolite catalyst]

The zeolite catalyst with a silica coating (hereinafter, also simply referred to as zeolite catalyst) to be obtained by the manufacturing method of the present embodiment is an unprecedented novel zeolite catalyst. Therefore, the present embodiment also includes a zeolite catalyst to be obtained by the above-described manufacturing method.

One of the characteristics of the zeolite catalyst of the present embodiment is the thinness of the silica coating. A silica coating thinner than that formed by the conventional method can be formed by manufacturing it by a manufacturing method different from the conventional method as described above, and it is considered that, as a result, the zeolite catalytic activity is improved and the yield in the manufacture of a compound using the catalyst is improved.

The thickness of the silica coating in the zeolite catalyst of the present embodiment is preferably 500 nm or less. A silica coating thin to such an extent could not be formed by the conventional manufacturing method. Owing to having a silica coating 500 nm or less in thickness, the zeolite catalyst can improve the yield in addition to attaining high isomer selectivity. When the thickness of the silica coating exceeds 500 nm, a sufficient yield cannot be obtained. There is no particular necessity to limit the lower limit of the thickness of the silica coating, but the thickness is usually 1 nm or more from the viewpoint of maintaining high isomer selectivity. The thickness of the silica coating is more preferably 5 nm or more and 50 nm or less.

The thickness of the silica coating is a value obtained by the method shown in Examples described later.

In the zeolite catalyst of the present embodiment, the amount of the silica coating is preferably 1 part by mass or more and 100 parts by mass or less per 100 parts by mass of the zinc-doped zeolite as a core catalyst. When the amount of the silica coating exceeds 100 parts by mass, there is a possibility that the yield when a compound is produced using the zeolite catalyst will be insufficient. In a more preferred embodiment, the amount of the silica coating is more desirably 3 parts by mass or more and 20 parts by mass or less per 100 parts by mass of the zinc-doped zeolite as a core catalyst.

The amount of the silica coating can be determined by a weight increase rate shown in Examples described later.

[Application of zeolite catalyst]

The zeolite catalyst of the present embodiment can be suitably used for the manufacture of an aromatic compound. Examples of the aromatic compound include benzene and aromatic hydrocarbons having a substituent such as toluene, xylene, and ethylbenzene, and in particular, the zeolite catalyst of the present embodiment is suitable for the manufacture of paraxylene. Paraxylene is a starting material for terephthalic acid to serve as a raw material for polyethylene terephthalate, and the catalyst of the present embodiment capable of manufacturing paraxylene in a high selectivity and a high yield is very useful for industrial use.

The method for manufacturing paraxylene using the zeolite catalyst of the present embodiment is not particularly limited, and paraxylene can be produced by reacting an aromatic hydrocarbon as a raw material with a methylating agent in the presence of the zeolite catalyst of the present embodiment.

Examples of the aromatic hydrocarbon as a raw material include benzene and alkylbenzenes. Examples of the methylating agent include methanol, dimethyl ether, dimethyl carbonate, and methane.

Although the present description discloses the techniques of various aspects as described above, the main technology among them is summarized below.

The method for manufacturing a zeolite catalyst relating to one aspect is a method for manufacturing a zeolite catalyst with a silica coating, the method including at least: mixing a zinc source and an MFI-type zeolite to provide a zinc-doped zeolite; preparing a mixture liquid containing a structure-directing agent containing tetraethylammonium, a silica source, and water; and adding the mixture liquid to a surface of the zinc-doped zeolite to perform hydrothermal synthesis.

According to the configuration described above, it is possible to provide a zeolite catalyst capable of obtaining a target compound from among isomers at a high ratio and in a high yield when manufacturing an aromatic compound having multiple isomers, such as xylene, and a method for manufacturing the catalyst.

In the method for manufacturing a zeolite catalyst according to a second aspect, in the manufacturing method of the first aspect, the silica coating is preferably a silicalite coating. This is advantageous in that the reaction on the outer surface of a crystal is inhibited.

In the method for manufacturing a zeolite catalyst according to a third aspect, in the manufacturing method of the first or second aspect, a weight increase rate of the zeolite catalyst with a silica coating with respect to the weight of the zinc-doped zeolite is 40% or less. Accordingly, it is considered that a thinner silica coating is formed in the resulting zeolite catalyst.

In the method for manufacturing a zeolite catalyst according to a fourth aspect, in the manufacturing method according to any one of the first to third aspects, the zeolite catalyst obtained by the manufacturing method is preferably a catalyst for paraxylene manufacture.

The zeolite catalyst for paraxylene manufacture according to a fifth aspect is a zeolite catalyst for paraxylene manufacture obtainable by the manufacturing method according to any one of the first to fourth aspects. The zeolite catalyst is suitable for the manufacture of paraxylene and is useful for industrial use.

**Examples**

Hereinafter, the present invention will be explained more specifically with reference to Examples, but the present invention is not limited at all by the Examples.

<Manufacture of zeolite catalyst>

(Example 1)

• Preparation of zinc-doped zeolite

Zeolite "HSZ-840HOA" (particle size: 3 μm) (1 g) manufactured by Tosoh Corporation as a core catalyst, 2 g of zinc nitrate hexahydrate manufactured by Nacalai Tesque, Inc. as a zinc source, and 66.5 g of distilled water were mixed, and the mixture was stirred at 80°C overnight. Thereafter, the mixture liquid was filtered by suction, followed by washing with 1 L of distilled water. Then, drying was performed in a dryer at 90°C approximately overnight until the liquid was thoroughly removed. The resulting dried matter was calcined at 550°C for 5 hours, providing a zinc-doped zeolite. At that time, the temperature was raised at 5°C/min, and was lowered by natural cooling. The resulting zinc-doped zeolite had an Si/Al content ratio of 16.4.

· Formation of silica coating

Tetraethyl orthosilicate (TEOS) (0.693 g) manufactured by FUJIFILM Wako Pure Chemical Corporation as a silica source, 0.28 g of a 35 wt% aqueous solution of tetraethylammonium hydroxide (TEAOH) manufactured by Sigma-Aldrich Co. LLC as a structure-directing agent, and 5.28 g of distilled water were placed in a Teflon (registered trademark) inner cylinder, and the mixture was stirred at room temperature for 1 hour, providing a mixture liquid containing the silica source, the structure-directing agent, and the water.
Into the resulting mixture liquid was added 0.3 g of the zinc-doped zeolite obtained above, and hydrothermal synthesis was performed for 24 hours under the conditions of a temperature of 180°C and 10 rpm in a rotary autoclave ("HYDROTHERMAL SYNTHESIS REACTION UNIT (device name)" manufactured by HIRO Company).
Thereafter, the supernatant was discarded, water was added thereto, and the mixture was centrifuged for 10 minutes. This operation was repeated three times to perform washing. Then, drying was performed in a dryer at 90°C approximately overnight until the liquid was thoroughly removed.
The resulting dried matter was calcined at 550°C for 5 hours, providing a zeolite catalyst with a silica coating. At that time, the temperature was raised at 5°C/min, and was lowered by natural cooling.

(Examples 2 to 4)

Zeolite catalysts with a silica coating were obtained in the same manner as in Example 1 except that the amount of the 35 wt% aqueous solution of tetraethylammonium hydroxide (TEAOH) as a structure-directing agent was changed to the amounts shown in Table 1 in the formation of the silica coating.

(Examples 5 to 7)

Zeolite catalysts with a silica coating were obtained in the same manner as in Example 1, except that in the formation of the silica coating, the amount of the 35 wt% aqueous solution of tetraethylammonium hydroxide (TEAOH) as a structure-directing agent was changed to the amounts shown in Table 1, and further, a 10 wt% aqueous solution of tetrapropylammonium hydroxide (TPAOH) manufactured by FUJIFILM Wako Pure Chemical Corporation was also added in the amounts shown in Table 1.

(Example 8)

A zeolite catalyst with a silica coating was obtained in the same manner as in Example 3 except that the amount of TEOS as a silica source and the amount of distilled water were changed to the amounts shown in Table 1 in the formation of the silica coating.

(Example 9)

A zeolite catalyst with a silica coating was obtained in the same manner as in Example 4 except that the amount of TEOS as a silica source and the amount of distilled water were changed to the amounts shown in Table 1 in the formation of the silica coating.

(Comparative Example)

· Preparation of zinc-doped zeolite

The preparation was carried out in the same manner as in Example 1.

· Formation of silica coating

Fumed silica (0.2 g) manufactured by Sigma-Aldrich Co. LLC as a silica source, 0.81 g of a 10 wt% aqueous solution of tetrapropylammonium hydroxide (TPAOH) manufactured by FUJIFILM Wako Pure Chemical Corporation as a structure-directing agent, 2.45 g of ethanol, and 13.6 g of distilled water were placed in an inner cylinder of Teflon (registered trademark; made by the inventors from a PTFE resin round bar), and the mixture was stirred at room temperature for 1 hour, providing a mixture liquid containing the silica source, the structure-directing agent, and the water.

Into the resulting mixture liquid was added 0.3 g of the zinc-doped zeolite obtained above, and hydrothermal synthesis was performed for 24 hours under the conditions of a temperature of 180°C and 10 rpm in a rotary autoclave ("HYDROTHERMAL SYNTHESIS REACTION UNIT (device name)" manufactured by HIRO Company).

Thereafter, the supernatant was discarded, water was added thereto, and the mixture was centrifuged for 10 minutes. This operation was repeated three times to perform washing. Then, drying was performed in a dryer at 90°C approximately overnight until the liquid was thoroughly removed.

The resulting dried matter was calcined at 550°C for 5 hours, providing a zeolite catalyst partially having a silica coating. At that time, the temperature was raised at 5°C/min, and was lowered by natural cooling.

In the zeolite catalyst obtained as described above, there still remained some portions uncoated with the silica coating, the above-described process of forming the silica coating was repeated from the beginning again. Specifically, a mixture liquid containing the silica source, the structure-directing agent, and water was prepared again, the zeolite catalyst obtained through the first calcination was added to the mixture liquid, and autoclaving, washing, drying, and calcination were performed under the same conditions as in the first calcination, providing a zeolite catalyst with a silica coating.

[Table 1]

|  | Zn-doped zeolite | TEOS | TEAOH (35 wt%) | TPAOH (10 wt%) | Distilled water |
|---|---|---|---|---|---|
|  | g | g | g | g | g |
| Example 1 | 0.3 | 0.693 | 0.28 | - | 5.28 |
| Example 2 | 0.3 | 0.693 | 0.42 | - | 5.28 |
| Example 3 | 0.3 | 0.693 | 0.56 | - | 5.28 |
| Example 4 | 0.3 | 0.693 | 1.12 | - | 5.28 |
| Example 5 | 0.3 | 0.693 | 0.14 | 0.14 | 5.28 |
| Example 6 | 0.3 | 0.693 | 0.28 | 0.14 | 5.28 |
| Example 7 | 0.3 | 0.693 | 0.14 | 0.28 | 5.28 |
| Example 8 | 0.3 | 1.386 | 0.56 | - | 10.56 |
| Example 9 | 0.3 | 1.386 | 1.12 | - | 10.56 |

<Evaluation method>

(Si/Al content ratio)

The content ratio of Si to Al (Si/Al ratio) in the zeolite catalyst a silica coating obtained in each of Examples and Comparative Example was analyzed by energy dispersive X-ray spectroscopy (EDX). The results are shown in Table 2.

(Weight increase rate due to silica coating formation and thickness of silica coating)

The zinc-doped zeolite as a core catalyst is composed of the elements of Si, Al, Zn and O, but the silica coating as a shell layer is composed of only Si and O. It was assumed that the contents of Zn and Al do not change during the synthesis of the coating. In addition, it was assumed that the weight of the catalyst is proportional to the volume (= the cube of the particle size). As the particle size of the core catalyst, the particle size of zeolite manufactured by Tosoh Corporation, namely 3 μm was adopted.

In the following formula, core (or core catalyst) denotes a zinc-doped zeolite, and core-shell (or core-shell catalyst) denotes a zeolite catalyst with a silica coating.

First, since the increment of the Si/Al ratio determined above is derived from Si contained in the silica coating, the weight increase rate (%) is calculated by the following Formula (1).

[Weight increase rate (%)] = ([core-shell Si/Al ratio] - [core Si/Al ratio])/([core Si/Al ratio] + 1) $\times$ 100          Formula (1):

In addition, since [weight increase rate (%)] = {[core-shell catalyst weight]/[core catalyst weight] - 1} $\times$ 100, the following Formula (2) is derived.

Formula (2):

$$[\text{Core-shell catalyst weight}]/[\text{core catalyst weight}] = [\text{weight increase rate (\%)}]/100 + 1$$

From the assumption described above, the weight of the catalyst is proportional to the volume (= the cube of the catalyst particle size), and thus the following Formula (3) is derived.

$$\{[\text{Core-shell catalyst particle size}]/[\text{core catalyst particle size}]\}^3 = [\text{core-shell catalyst weight}]/[\text{core catalyst weight}]$$ Formula (3):

The thickness of the silica coating can be calculated by the following formula (4) from the difference in catalyst particle size between the core-shell catalyst and the core catalyst.

$$[\text{Thickness of silica coating}] = \{[\text{core-shell catalyst particle size}] - [\text{core catalyst particle size}]\}/2 = [\text{core catalyst particle size}] \times \{([\text{core-shell catalyst particle size}]/[\text{core catalyst particle size}]) - 1\}/2$$ Formula (4):

From the assumption, Formula (5): the core catalyst particle size = 3 μm.

In addition, regarding the core-shell catalyst particle size in the above Formulas (3) and (4), Formula (3) is substituted into Formula (4) to form the following Formula (6):

$$[\text{Thickness of silica coating}] = [\text{core catalyst particle size}] \times \{ [\text{core-shell catalyst weight}]/[\text{core catalyst weight}]\}^{1/3} - 1 \}/2$$

and the "core-shell catalyst particle size" is offset, so that the thickness can be calculated from the catalyst weight ratio and the core particle size.

The thickness of the silica coating was calculated by substituting the above Formulas (1), (2), and (5) into Formula (6). The results are shown in Table 2.

[Table 2]

| | | Zn-doped zeolite | TEOS | TEAOH (35 wt%) | TPAOH (10 wt%) | Distilled water | Si/Al ratio | Weight increase rate | Thicknes s of coating |
|---|---|---|---|---|---|---|---|---|---|
| | | g | g | g | g | g | - | % | nm |
| | Example 1 | 0.3 | 0.693 | 0.28 | - | 5.28 | 17.7 | 7.6 | 37 |
| | Example 2 | 0.3 | 0.693 | 0.42 | - | 5.28 | 17.9 | 8.5 | 41 |
| | Example 3 | 0.3 | 0.693 | 0.56 | - | 5.28 | 17.8 | 7.9 | 39 |
| | Example 4 | 0.3 | 0.693 | 1.12 | - | 5.28 | 36.6 | 116 | 439 |
| | Example 5 | 0.3 | 0.693 | 0.14 | 0.14 | 5.28 | 26.5 | 58 | 248 |
| | Example 6 | 0.3 | 0.693 | 0.28 | 0.14 | 5.28 | 34.6 | 104 | 403 |
| | Example 7 | 0.3 | 0.693 | 0.14 | 0.28 | 5.28 | 33.6 | 99 | 387 |
| | Example 8 | 0.3 | 1.386 | 0.56 | - | 10.56 | 17.4 | 5.8 | 29 |
| | Example 9 | 0.3 | 1.386 | 1.12 | - | 10.56 | 17.4 | 5.8 | 29 |
| | Comparativ e Example | 0.3 | - | - | - | - | 50.9 | 198 | 659 |

(Catalyst performance)

Paraxylene was produced by the following method using one of the zeolite catalysts of Examples 1 to 5 and 8 and Comparative Example, and the paraxylene yield and the paraxylene selectivity of each catalyst were evaluated.

· Manufacture of paraxylene

A fixed-bed reactor was filled with 0.05 g of a zeolite catalyst, and a reaction was carried out by feeding methanol gas at 400°C under atmospheric pressure. The generated gas after 20 minutes from the start of the reaction was analyzed and the catalytic activity was evaluated.

· Method for analyzing generated gas

The analysis was performed with the following analyzer and conditions:

Analyzer: GC-2014 manufactured by Shimadzu Corporation
Column: capillary column Xylene Master
Initial column temperature: 70°C
Heating rate: 2°C/min
Detector temperature: 200°C

· Methods for calculating yield and paraxylene selectivity

The yield and the paraxylene selectivity were determined by the following formulas:

[Paraxylene yield (C mol%)] = [amount of paraxylene produced (mol)]/[amount of methanol fed (mol)]/8 × 100 [Paraxylene selectivity (%)] = [amount of paraxylene produced (mol)]/[amount of xylene produced (total of ortho, meta, and para) (mol)] × 100

The evaluation results are shown in Table 3.

[Table 3]

|  | Si/Al ratio | Weight increase rate | Thickness of coating | Yield | Para selectivity |
|---|---|---|---|---|---|
|  | - | % | nm | C-mol% | % |
| Example 1 | 17.7 | 7.6 | 37 | 16 | 99 |
| Example 2 | 17.9 | 8.5 | 41 | 17 | 99 |
| Example 3 | 17.8 | 7.9 | 39 | 16 | 99 |
| Example 4 | 36.6 | 116 | 439 | 15 | 99 |
| Example 5 | 26.5 | 58 | 248 | 16 | 98 |
| Example 8 | 17.4 | 5.8 | 29 | 16 | 99 |
| Comparativ e Example | 50.9 | 198 | 659 | 10 | 99 |

(Discussion)

From the results in Table 2, it was confirmed that the zeolite catalysts obtained by the manufacturing methods of Examples were smaller in the thickness of silica coating than the zeolite catalyst obtained by the manufacturing method of Comparative Example, which is a conventional method. This is also supported by the weight increase rate after silica coating formation. From the results in Table 3, it was confirmed that the zeolite catalysts obtained by the manufacturing methods of Examples had a silica coating 500 nm or less in thickness, the yields in paraxylene manufacture were high, and the paraxylene selectivities were also high.

On the other hand, it is found that in the zeolite catalyst obtained by the manufacturing method of Comparative Example, the thickness of the silica coating exceeded 500 nm, and the paraxylene selectivity in the manufacture of paraxylene was high, but the yield was lower than that of the zeolite catalysts of Examples.

This application is based on Japanese Patent Application No. 2022-58510 filed on March 31, 2022, the contents of which are included in the present application.

To embody the present invention, the present invention has been appropriately and sufficiently described through the embodiments with reference to the specific examples and the drawings in the above, but it should be recognized that those skilled in the art can easily modify and/or improve the above-described embodiments. Therefore, as long as modifications or improvements carried out by a person skilled in the art do not depart from the scope of the claims described in the patent claims, these modifications or improvements are interpreted as being encompassed by the scope of the claims.

**Claims**

1. A method for manufacturing a zeolite catalyst with a silica coating, the method comprising at least:

   mixing a zinc source and an MFI-type zeolite to obtain a zinc-doped zeolite;
   preparing a mixture liquid containing a structure-directing agent containing tetraethylammonium, a silica source, and water; and
   adding the mixture liquid to a surface of the zinc-doped zeolite to perform hydrothermal synthesis.

2. The method for manufacturing a zeolite catalyst according to claim 1, wherein the silica coating is a silicalite coating.

3. The method for manufacturing a zeolite catalyst according to claim 1, wherein a weight increase rate of the zeolite catalyst with a silica coating with respect to the weight of the zinc-doped zeolite is 40% or less.

4. The method for manufacturing a zeolite catalyst according to claim 1, wherein the zeolite catalyst is a catalyst for paraxylene manufacture.

5. A zeolite catalyst for paraxylene manufacture, obtainable by the method according to any one of claims 1 to 4.

# FIG.1

## INTERNATIONAL SEARCH REPORT

| International application No. |
|---|
| **PCT/JP2023/013080** |

**A. CLASSIFICATION OF SUBJECT MATTER**

*B01J 29/40*(2006.01)i; *C01B 39/36*(2006.01)i; *C07C 1/20*(2006.01)i; *C07C 15/08*(2006.01)i; *C07B 61/00*(2006.01)n
FI: B01J29/40 Z; C01B39/36; C07C1/20; C07C15/08; C07B61/00 300

According to International Patent Classification (IPC) or to both national classification and IPC

**B. FIELDS SEARCHED**

Minimum documentation searched (classification system followed by classification symbols)

B01J29/40; C01B39/36; C07C1/20; C07C15/08; C07B61/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | CN 104549479 A (CHINA PETROLEUM & CHEMICAL CORPORATION) 29 April 2015 (2015-04-29)<br>paragraphs [0032], [0036]-[0050] | 1-2 |
| A | paragraphs [0032], [0036]-[0050] | 3-5 |
| A | CN 109675617 A (CHINA PETROLEUM & CHEMICAL CORPORATION) 26 April 2019 (2019-04-26)<br>paragraphs [0037]-[0043] | 1-5 |
| A | JP 2019-205969 A (NIPPON STEEL CORP) 05 December 2019 (2019-12-05)<br>paragraphs [0027]-[0040], [0057], [0066] | 1-5 |
| A | JP 2010-208948 A (MITSUI CHEMICALS INC) 24 September 2010 (2010-09-24)<br>paragraphs [0031]-[0033] | 1-5 |
| A | CN 111420632 A (CHINA UNIVERSITY OF PETROLEUM, BEIJING) 17 July 2020 (2020-07-17)<br>paragraphs [0116]-[0147] | 1-5 |

☐ Further documents are listed in the continuation of Box C.  ☑ See patent family annex.

| * Special categories of cited documents: | |
|---|---|
| "A" document defining the general state of the art which is not considered to be of particular relevance | "T" later document published after the international filing date or priority date and not in conflict with the application but cited to understand the principle or theory underlying the invention |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **02 June 2023** | **20 June 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT
### Information on patent family members

International application No.

**PCT/JP2023/013080**

| Patent document cited in search report | | | Publication date (day/month/year) | Patent family member(s) | Publication date (day/month/year) |
|---|---|---|---|---|---|
| CN | 104549479 | A | 29 April 2015 | (Family: none) | |
| CN | 109675617 | A | 26 April 2019 | (Family: none) | |
| JP | 2019-205969 | A | 05 December 2019 | (Family: none) | |
| JP | 2010-208948 | A | 24 September 2010 | (Family: none) | |
| CN | 111420632 | A | 17 July 2020 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010208948 A

- JP 2022058510 A